# EUROPÄISCHE PATENTSCHRIFT

(11) **EP 0 823 849 B1**
(45) Veröffentlichungstag und Bekanntmachung des Hinweises auf die Patenterteilung: **22.12.1999**
(21) Anmeldenummer: 96910852.1
(22) Anmeldetag: 30.04.1996
(51) Int. Cl.: A61M 16/00

(54) **VORRICHTUNG ZUR ABGABE EINES BEATMUNGSGASES**
DEVICE FOR ADMINISTERING A GAS TO BE INHALED
DISPOSITIF D'ADMINISTRATION D'UN GAZ A INHALER

(30) Priorität: 02.05.1995 AT 24395
(43) Veröffentlichungstag der Anmeldung: 18.02.1998
(73) Patentinhaber: Aloy, Alexander, Dr., 3400 Klosterneuburg (AT)
(72) Erfinder: ALOY, Alexander, A-3400 Klosterneuburg (AT); SCHRAGL, Eva, A-1090 Wien (AT)
(74) Vertreter: Haffner, Thomas M., Dr.
(86) Internationale Anmeldenummer: AT9600086
(87) Internationale Veröffentlichungsnummer: WO9634643

(56) Entgegenhaltungen:
- EP-A- 0 080 155
- EP-A- 0 149 722
- EP-A- 0 153 991
- EP-A- 0 234 736
- GB-A- 2 033 759

## Beschreibung

Die Erfindung bezieht sich auf eine Vorrichtung zur Abgabe eines Beatmungsgases mit einem T-Konnektor, an dessen T-Balken ein Atemgasquerstrom angeschlossen ist, wobei quer zum Balken (1) des T-Konnektors (2) ein Rohr mit geringerem lichten Querschnitt als der lichte Querschnitt des quer zum T-Balken (1) anschließenden rohrförmigen Teiles (3) des T-Konnektors angeordnet ist, welches sich in Richtung des quer zum T-Balken (1) verlaufenden rohrförmigen Teiles (3) des T-Konnektors (2) erstreckt. Eine derartige Vorrichtung ist schon aus der EP-A-0 234 736 bekannt.

Das ARDS (adult respiratory distress syndrom) ist ein Lungenversagen beim Erwachsenen, welches multifaktoriell ausgelöst durch eine schwere Störung des Gasaustausches in der Lunge gekennzeichnet ist. Die Behandlung dieser respiratorischen Insuffizienz erfolgt mittels maschineller künstlicher Beatmung. Seit Jahren gibt es immer wieder Versuche, das Beatmungsgas mit hohem Ausgangsdruck und hoher Beatmungsfrequenz (100-1500/min) über eine Düse mit kleinem Querschnitt durch einen Beatmungstubus in die Lunge zu verabreichen (Jet-Ventilation).

Beispielsweise ist aus der EP 0 234 736 A1 ein Jet-Ventilation-System bekanntgeworden, bei welchem ein an den T-Balken eines im wesentlichen T-förmigen Konnektors angeschlossener Atemgasstrom und ein sich in Richtung des quer zum T-Balken verlaufenden rohrförmigen Teiles des Konnektors erstreckendes düsenförmiges Rohr, über welches ein mit einstellbarer Frequenz pulsierendes Beatmungsgas eingebracht wird, vorgesehen sind.

Die GB 2 033 759 A sowie die EP 0 153 991 A1 beschreiben eine Vorrichtung zur pulsierenden Abgabe eines Beatmungsgases mit einer Mehrzahl an Druckgasrohren.

Die Erfindung zielt nun darauf ab, eine Vorrichtung der eingangs genannten Art dahingehend weiterzubilden, daß gleichzeitig mit der Grundbeatmung ein ausreichender Spitzendruck bzw. Druckplateau bedingt durch eine niedere Beatmungsfrequenz mit hohem Auslung mit Luft, dem endexpiratorischen Druckplateau, erzeugt durch eine hohe Beatmungsfrequenz mit ebenfalls hohem Ausgangsdruck, sichergestellt ist, um ein Kollabieren der Lunge mit Sicherheit zu verhindern. Gleichzeitig soll die erfindungsgemäße Einrichtung auch weitere Funktionen ermöglichen und insbesondere eine sichere Messung des durch die Gasgemische erzeugten Beatmungsdruckes in der Lunge gewährleisten. Schließlich soll erfindungsgemäß die Möglichkeit geschaffen werden, auch weitere therapeutische oder diagnostische Maßnahmen ohne Wechsel der Vorrichtung durchführen zu können.

Zur Lösung dieser Aufgabe besteht die erfindungsgemäße Ausbildung im wesentlichen darin, daß quer zum Balken des T-Konnektors eine Mehrzahl von Rohren mit geringerem lichten Querschnitt als dem lichten Querschnitt des quer zum T-Balken anschließenden rohrförmigen Teiles des T-Konnektors angeordnet sind, welche sich in Richtung des quer zum T-Balken verlaufenden rohrförmigen Teilesdes T-Konnektors erstrecken, wobei wenigstens zwei Rohre mit pulsierendem Druckgas unterschiedlicher Frequenz beaufschlagt sind. Dadurch, daß eine Mehrzahl von Rohren mit geringerem lichten Querschnitt zum Einsatz gelangen, ist es nun möglich, nicht nur Beatmungsgas mit hoch vorbestimmtem Druck und vorgegebener Beatmnungsfrequenz, sondern gleichzeitig unterschiedliche Beatmungsfrequenzen über unterschiedliche, von einander verschiedene Rohre vorzugebenen. Eine derartige Verwendung von zwei voneinander verschiedenen Beatmungsfrequenzen sichert einesteils die entsprechende Basisfüllung während der Expiration (positiv endexpiratorischer Druck) und verhindert somit ein vollständiges Kollabieren der Lunge (hohe Beatmungsfrequenz), und ermöglicht andererseits eine entsprechende Aufblähung (inspiratorisches Druckplateau) während der Inspiration (niedere Beatmungsfrequenz). Durch weitere, derartige Rohre mit geringerem lichten Querschnitt läßt sich der durch die Gasgemische erzeugte Beatmungsdruck in der Lunge mit Sicherheit an der hierfür geeigneten Stelle messen, wobei gleichzeitig über ein weiteres derartiges Rohr beispielsweise isotonische Kochsalzlösung oder therapeutisch wirksame Substanzen eingedüst und zerstäubt werden können. Mit Vorteil ist die Einrichtung derart weitergebildet, daß die Rohre in axialer Richtung unterschiedliche Länge aufweisen und daß das die größte axiale Länge aufweisende Rohr mit einer Druckmeßeinrichtung verbunden ist. Durch die Messung des Druckes über das die größte axiale Länge aufweisende Rohr wird ein Druck nahe der Lunge erfaßt und damit tatsächlich eine sichere Registrierung des Beatmungsdruckes ermöglicht.

Um therapeutisch wirksame Substanzen bzw. die Respiration begünstigende Bedingungen zu gewährleisten, ist mit Vorteil die Ausbildung so getroffen, daß ein Rohr mit einer im wesentlichen radialen Austrittsdüse ausgebildet ist und daß der Austrittsdüse benachbart ein kürzeres Rohr mündet. Über ein derartiges Rohr mit einer im wesentlichen radialen Austrittsdüse kann Kochsalzlösung geführt werden und an der Austrittsdüse durch das benachbarte kürzere Rohr zerstäubt werden. Mit Vorteil ist hierbei die Ausbildung so getroffen, daß das mit höherer Gaspulsfrequenz beaufschlagte Rohr nahe der radialen Austrittsdüse mündet und axial länger ausgebildet ist als das Rohr für pulsierendes Druckgas geringerer Frequenz, wodurch eine besonders gute Zerstäubung gewährleistet wird.

Um weitere diagnostische Maßnahmen zu ermöglichen und insbesondere beispielsweise die Durchführung einer Bronchoskopie zu erlauben, ist mit Vorteil die Ausbildung so getroffen, daß an den T-Konnektor ein verschließbarer Trichter mündet.

Ein besonders einfacher Zusammenbau der erfindungsgemäßen Vorrichtung und ein einfacher Aufbau kann dadurch erzielt werden, daß die Rohre in einem Ansatzstück festgelegt sind, welches dichtend mit dem T-Konnektor verriegelbar ist, wobei mit Vorteil das Ansatzstück als Deckel ausgebildet ist, welcher mit einer mit dem auf den T-Balken normalen, rohrförmigen Teil des T-Konnektors fluchtenden Öffnung des T-Balkens des T-Konnektors über ein Bajonett verriegelbar ist. Ein derartiger Deckel läßt sich einfach an der entsprechenden Öffnung aufsetzen und verriegeln, wobei die Fertigung des die Mehrzahl von Rohren tragenden Deckels in besonders einfacher Weise vorgenommen werden kann.

Um an die unterschiedlichen Rohre entsprechend Gas oder Flüssigkeitsquellen in einfacher und standardisierter Weise anschließen zu können, ist mit Vorteil die Ausbildung so getroffen, daß die Rohre an den oberhalb des T-Balkens des T-Konnektors liegenden Enden Standardanschlußstücke wie z.B. Lueranschlüsse tragen.

Eine besonders einfache und betriebssichere Einrichtung läßt sich dadurch sicherstellen, daß vier Rohre im Ansatzstück festgelegt sind, deren Gesamtquerschnitt kleiner als 25 %, vorzugsweise kleiner als 10 %, des lichten Querschnittes des auf den T-Balken normalen, rohrförmigen Teiles des T-Konnektors ist, wobei mit einer derartigen Einrichtung neben der kontinuierlichen Überwachung des Beatmungsdruckes Kochsalzlösung versprüht werden kann, und gleichzeitig mit hoher und mit geringerer Frequenz, beispielsweise mit einem Beatmungsgas unter einem Druck von etwa 1,5 bis 3 bar, beispielsweise 2 bar, gearbeitet werden kann.

In einer weiterführenden Ausbildung wird die Funktionssicherheit der erfindungsgemäßen Vorrichtung vorteilhaft dadurch weiter erhöht, daß nunmehr die sonst ungeschützten proximalen Rohrabschnitte verstärkt ausgebildet sind, was sich beispielsweise durch zu den Rohren Koaxiale Spiralfedern erreichen läßt, werden die Knick- und Torsionsfestigkeit erhöht. Die hiermit erzielte große Widerstandsfähigkeit der Rohrwände kommt insbesondere dann zum Tragen, wenn die Vorrichtung notfallbedingt höheren Beanspruchungen ausgesetzt ist.

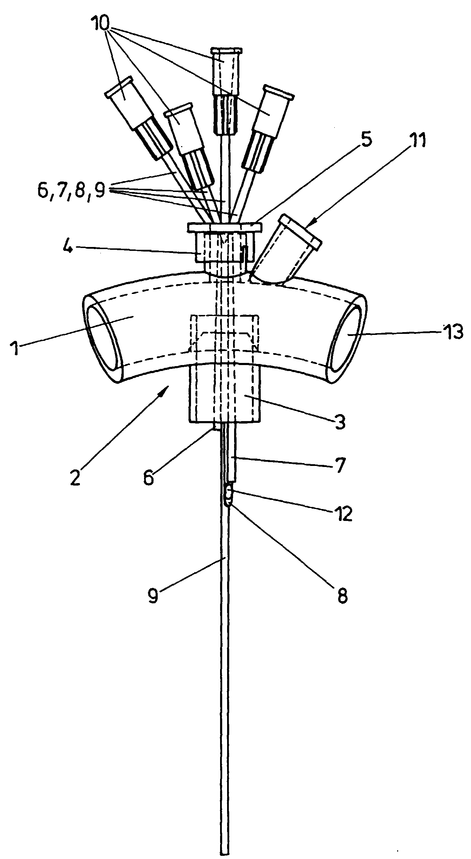

Die erfindungsgemäße Einrichtung wird nachfolgend anhand eines in der Zeichnung schematisch dargestellten Ausführungsbeispieles näher erläutert.

In der Zeichnung ist mit 1 der T-Balken eines T-Konnektors 2 bezeichnet. An den T-Balken 1 schließt ein sich quer zum T-Balken erstreckender rohrförmiger Teil 3 an. An der gegenüberliegenden Seite des T-förmigen Balkens 1 ist eine Öffnung 4 vorgesehen, welche mit der Achse des an den T-Balken 1 anschließenden Rohres fluchtet.

Auf die Öffnung 4 ist ein Deckel 5 aufgesetz, welcher vier Rohre mit gegenüber dem lichten Querschnitt des Rohres 3 wesentlich geringerem Querschnitt 6, 7, 8 und 9 trägt. An diese Rohre 6, 7, 8 und 9 sind Lueranschlüsse 10 angeschlossen.

An den T-förmigen Balken 1 ist schließlich ein verschließbarer Trichter 11 angeschlossen, über welchen eine Bronchoskopie möglich wird. Die Rohre 5, 7, 8 und 9 weisen unterschiedliche axiale Länge auf. Das kürzeste Rohr 6 ist mit Beatmungsgas geringer Frequenz beaufschlagt. Das eine radiale Austrittsöffnung 12 aufweisende Rohr 8 weist seine radiale Austrittsöffnung nahe der Mündung des entsprechend kürzeren Rohres 7 auf. Über das Rohr 7 wird hierbei Beatmungsgas mit höherer Beatmungsfrequenz zur Zerstäubung von über das Rohr 8 zugeführten flüssigen Medien eingebracht. Das in axialer Richtung längste Rohr 9 dient der kontinuierlichen Druckmessung.

An den T-Balken 1 ist ein Atemgasquerstrom angeschlossen, wobei über 13 die Gaszufuhr eines befeuchteten und erwärmten Gases erfolgt, und die Expiration über die Öffnung 14 erfolgt.

## Patentansprüche

1. Vorrichtung zur Abgabe eines Beatmungsgases mit einem T-Konnektor (2), an dessen T-Balken (1) ein Atemgasquerstrom angeschlossen ist, wobei quer zum Balken (1) des T-Konnektors (2) eine Mehrzahl von Rohren (6, 7, 8, 9) mit geringerem lichten Querschnitt als dem lichten Querschnitt des quer zum T-Balken (1) anschließenden rohrförmigen Teiles (3) des T-Konnektors (2) angeordnet sind, welche sich in Richtung des quer zum T-Balken (1) verlaufenden rohrförmigen Teiles (3) des T-Konnektors (2) erstrecken, wobei wenigstens zwei Rohre (6, 7) mit pulsierendem Druckgas unterschiedlicher Frequenz beaufschlagt sind.

2. Vorrichtung nach Anspruch 1, dadurch gekennzeichnet, daß die Rohre (6, 7, 8, 9) in axialer Richtung unterschiedliche Länge aufweisen und daß das die größte axiale Länge aufweisende Rohr (9) mit einer Druckmeßeinrichtung verbunden ist.

3. Vorrichtung nach Anspruch 1 oder 2, dadurch gekennzeichnet, daß ein Rohr (8) mit einer im wesentlichen radialen Austrittsdüse (12) ausgebildet ist und daß der Austrittsdüse (12) benachbart ein kürzeres Rohr (7) mündet.

4. Vorrichtung nach Anspruch 3, dadurch gekennzeichnet, daß das mit höherer Gaspulsfrequenz beaufschlagte Rohr (7) nahe der radialen Austrittsdüse (12) mündet und axial länger ausgebildet ist als das Rohr (6) für pulsierendes Druckgas geringerer Frequenz.

5. Vorrichtung nach einem der Ansprüche 1 bis 4, dadurch gekennzeichnet, daß an den T-Konnektor (2) ein verschließbarer Trichter (11) mündet.

6. Vorrichtung nach einem der Ansprüche 1 bis 5, dadurch gekennzeichnet, daß die Rohre (6, 7, 8, 9) in einem Ansatzstück (5) festgelegt sind, welches dichtend mit dem T-Konnektor (2) verriegelbar ist.

7. Vorrichtung nach Anspruch 6, dadurch gekennzeichnet, daß das Ansatzstück (5) als Deckel ausgebildet ist, welcher mit einer mit dem auf den T-Balken (1) normalen, rohrförmigen Teil des T-Konnektors fluchtenden Öffnung (4) des T-Balkens (1) des T-Konnektors (2) über ein Bajonett verriegelbar ist.

8. Vorrichtung nach einem der Ansprüche 1 bis 7, dadurch gekennzeichnet, daß die Rohre (6, 7, 8, 9) an den oberhalb des T-Balkens (1) des T-Konnektors (2) liegenden Enden Standardanschlußstücke wie z.B. Lueranschlüsse (10) tragen.

9. Vorrichtung nach einem der Ansprüche 1 bis 8, dadurch gekennzeichnet, daß vier Rohre (6, 7, 8, 9) im Ansatzstück (5) festgelegt sind, deren Gesamtquerschnitt kleiner als 25 %, vorzugsweise kleiner als 10 %, des lichten Querschnittes des auf den T-Balken (1) normalen, rohrförmigen Teiles (3) des T-Konnektors ist.

10. Vorrichtung nach einem der Ansprüche 1 bis 9, dadurch gekennzeichnet, daß die vier Rohre (6,7,8,9) im proximalen Bereich verstärkt ausgebildet sind.

## Claims

1. Apparatus for delivering respiratory gas with a T connector (2) to the crosspiece (1) of which a breathing gas crossflow is connected, a plurality of tubes (6, 7, 8, 9) of smaller internal cross-section than the internal cross-section of the tubular part (3) of the T connector (2) connected transversely to the crosspiece (1) being themselves disposed transversely to the crosspiece (1) and extending in the direction of the tubular part (3) of the T connector (2) extending transversely to the crosspiece (1), at least two tubes (6, 7) being supplied with compressed gas pulsating at different frequencies.

2. Apparatus according to Claim 1, characterized in that the tubes (6, 7, 8, 9) are of different lengths in the axial direction, and in that the tube (9) with the greatest axial length is connected to a manometer.

3. Apparatus according to Claim 1 or Claim 2, characterized in that a tube (8) is formed with an essentially radial outlet nozzle (12) and in that a shorter tube (7) has its terminal opening adjacent to the outlet nozzle (12).

4. Apparatus according to Claim 3, characterized in that the tube (7) supplied at the higher gas pulsation frequency has its terminal opening close to the radial outlet nozzle (12) and is axially longer than the tube (6) for compressed gas pulsating at the lower frequency.

5. Apparatus according to any one of Claims 1 to 4, characterized in that a stoppable mouth (11) opens into the T connector (2).

6. Apparatus according to any one of Claims 1 to 5, characterized in that the tubes (6, 7, 8, 9) are fixed in an attachment piece (5) which can be locked onto the T connector (2) in a gastight manner.

7. Apparatus according to Claim 6, characterized in that the attachment piece (5) is formed as a cap which can be locked by a bayonet type coupling onto an orifice (4) of the crosspiece (1) of the T connector (2) said orifice (4) being aligned with the tubular part of the T connector perpendicular to the crosspiece (1).

8. Apparatus according to any one of Claims 1 to 7, characterized in that the tubes (6, 7, 8, 9) carry standard connection pieces such as e.g. Luer connections (10) at the ends located above the crosspiece (1) of the T connector (2).

9. Apparatus according to any one of Claims 1 to 8, characterized in that four tubes (6, 7, 8, 9) are fixed in the attachment piece (5), their total area of cross-section being less than 25%, preferably less than 10%, of the internal cross-section of the tubular part (3) of the T connector perpendicular to the crosspiece (1).

10. Apparatus according to any one of Claims 1 to 9, characterized in that the four tubes (6, 7, 8, 9) are formed with thicker walls in the proximal region.

## Revendications

1. Dispositif de distribution d'un gaz de ventilation artificielle, comprenant un connecteur en T (2), à la barre de T (1) duquel est relié un courant transversal de gaz ventilatoire, une pluralité de tubes (6, 7, 8, 9) à section intérieure inférieure à la section intérieure de la partie tubulaire (3) du connecteur en T (2) raccordée transversalement à la barre de T (1) étant disposée transversalement à la barre (1) du connecteur en T (2), lesdits tubes s'étendant en direction de la partie tubulaire (3) du connecteur en T (2) qui est transversale à la barre de T (1), au moins deux tubes (6, 7) étant alimentés en gaz sous pression pulsé à des fréquences différentes.

2. Dispositif selon la revendication 1, caractérisé en ce que les tubes (6, 7, 8, 9) présentent des longueurs différentes dans la direction axiale, et en ce que le tube (9) présentant la longueur axiale la plus grande est relié à un dispositif de mesure de pression.

3. Dispositif selon la revendication 1 ou 2, caractérisé en ce qu'un tube (8) est pourvu d'une buse de sortie sensiblement radiale (12), et en ce qu'un tube plus court (7) débouche au voisinage de la buse de sortie (12).

4. Dispositif selon la revendication 3, caractérisé en ce que le tube (7) alimenté à une fréquence de pulsation de gaz plus élevée débouche près de la buse de sortie radiale (12) et est axialement plus long que le tube (6) destiné à du gaz sous pression pulsé à fréquence plus faible.

5. Dispositif selon l'une des revendications 1 à 4, caractérisé en ce qu'un entonnoir obturable (11) débouche dans le connecteur en T (2).

6. Dispositif selon l'une des revendications 1 à 5, caractérisé en ce que les tubes (6, 7, 8, 9) sont immobilisés dans un embout (5) qui peut être verrouillé de manière étanche sur le connecteur en T (2).

7. Dispositif selon la revendication 6, caractérisé en ce que l'embout (5) est conçu sous la forme d'un couvercle qui, par l'intermédiaire d'une liaison à baïonnette, peut être verrouillé sur l'orifice (4) de la barre de T (1) du connecteur en T (2), ledit orifice étant aligné avec la partie tubulaire du connecteur en T qui est perpendiculaire à la barre (1) du T.

8. Dispositif selon l'une des revendications 1 à 7, caractérisé en ce que les tubes (6, 7, 8, 9) portent des raccords, comme par exemple des raccords de Luer (10), à leur extrémité située au-dessus de la barre de T (1) du connecteur en T (2).

9. Dispositif selon l'une des revendications 1 à 8, caractérisé en ce que dans l'embout (5) sont immobilisés quatre tubes (6, 7, 8, 9) dont la section totale est inférieure à 25 %, de préférence inférieure à 10 %, de la section intérieure de la partie tubulaire (3) du connecteur en T perpendiculaire à la barre (1) du T.

10. Dispositif selon l'une des revendications 1 à 9, caractérisé en ce que les quatre tubes (6, 7, 8, 9) sont renforcés dans la zone proximale.
